Europäisches Patentamt

European Patent Office

Office européen des brevets

(19)

(11) Veröffentlichungsnummer: **0 205 643 B1**

## (12) EUROPÄISCHE PATENTSCHRIFT

(45) Veröffentlichungstag der Patentschrift: **13.03.91**

(21) Anmeldenummer: **85107836.0**

(22) Anmeldetag: **25.06.85**

(51) Int. Cl.5: **G01N 33/53**, G01N 33/545, //G01N33/564

(54) **Immuntest zum Nachweis von Antikörpern gegen DNS.**

(43) Veröffentlichungstag der Anmeldung:
**30.12.86 Patentblatt 86/52**

(45) Bekanntmachung des Hinweises auf die Patenterteilung:
**13.03.91 Patentblatt 91/11**

(84) Benannte Vertragsstaaten:
**AT BE CH DE FR GB IT LI LU NL SE**

(56) Entgegenhaltungen:
EP-A- 0 101 912
EP-A- 0 134 307
EP-A- 0 135 051
EP-A- 0 135 159
DE-A- 2 738 183

CHEMICAL ABSTRACTS, Band 101, Nr. 21, 19 November 1984, Columbus, OH (US); S. 580, Nr. 189598u

CHEMICAL ABSTRACTS, Band 99, Nr. 25, 19 Dezember 1983, Columbus, OH (US); R.L.RUBIN et al., S. 505, Nr. 210885n

JOURNAL OF IMMUNOLOGOCAL METHODS, Band 63(3), 1983; Seiten 359-366

(73) Patentinhaber: **PROGEN Biotechnik GmbH**
**Im Neuenheimer Feld 519**
**W-6900 Heidelberg(DE)**

(72) Erfinder: **Rauterberg, Ernst W., Dr., c/o**
**Inst.f.Immunpathol.**
**Ruprecht Karls-Univ., Im Neuenheimer Feld**
**305**
**W-6900 Heidelberg(DE)**

(74) Vertreter: **Hach, Hans Karl, Dr.**
**Tarunstrasse 23**
**W-6950 Mosbach-Waldstadt(DE)**

## Beschreibung

Antikörper gegen Doppelstrang-DNS können grundsätzlich in zwei Gruppen eingeteilt werden, nämlich in Antikörper, die nur mit natürlicher DNS in seiner Helixkonfiguration reagieren und somit keine Kreuzreaktion mit Einzelstrang-DNS zeigen und Antikörper, die sowohl mit Doppelstrang-DNS als auch mit Einzelstrang-DNS reagieren.

Beide genannten Antikörpergruppen sind insbesondere beim systemischen Lupus erythematosus (SLE) signifikant gehäuft; insbesondere höhere Titer von Autoantikörpern gegen Doppelstrang-DNS kommen fast ausschließlich bei der genannten Systemerkrankung SLE vor. Der Nachweis von Autoantikörpern gegen Doppelstrang-DNS stellt somit einen der validesten Laborparameter bei der Diagnose dieser Erkrankung dar. Der Nachweis von Autoantikörpern gegen Doppelstrang-DNS ist insbesondere von differentialdiagnostischer Bedeutung bei der Abgrenzung des SLE von anderen Autoantikörperkrankheiten (Kollagenosen).

In der Literatur wurden mehrere Methoden beschrieben, mit denen der Nachweis von Autoantikörpern gegen Doppelstrang-DNS erfolgt.

Eine Methode ist die passive Hämagglutination nach Kupplung von Kalbsthymus-DNS an chromierte Schafserythrozyten (Sasaki et al., 1982, Tohoko J. exp. Med. 38 : 341) durch einen Bentonit-Flokulationstest (Kayhoe et al. 1960, Nej Med. 263 : 5). Bei der passiven Hämagglutination wurde jedoch nicht getestet, inwieweit nur Doppelstrang-DNS an die chromierten Schafserythrozyten bindet. Da bei den passiven Hämagglutinationstests überdies nur mit Verdünnungen gearbeitet werden kann, sind sie verhältnismäßig ungenau.

Es wurden weiterhin Radioimmunoassays zum Nachweis von Autoantikörpern gegen Doppelstrang-DNS beschrieben, die auf der von Farr erstmals beschriebenen Methode (Farr 1958, J. Infect. Dis. 193: 239) basieren. Hierbei wird eine Patientenserumprobe mit radioaktiv markierter Doppelstrang-DNS inkubiert, anschließend wird ein gleiches Volumen an gesättigtem Ammoniumsulfat hinzugefügt. Dabei präzipitieren die Gamma-Globuline, während die markierte DNS normalerweise in Lösung bleibt. Falls die Serumprobe Antikörper gegen DNS enthält, wird diese markierte DNS mitpräzipitiert. Dies läßt sich anschließend durch Messung der Radioaktivität in dem Präzipitat messen.

Diese Methode ist die Basis zu einer von vier käuflichen Testkits zum Nachweis von Doppelstrang-DNS-Antikörpern. Es handelt sich dabei um die Produkte der Fa. Amersham Buchler sowie den LUPO-TEC-Radioimmunoassay der Fa. Wampole Laboratories, Cranbury. Der Amersham-Assay hat in Deutschland nahezu eine Monopolstellung inne. Er wird von zahlreichen niedergelassenen Laborärzten und Krankenhauslaboratorien benutzt. Im Gegensatz zu dem ursprünglichen Farr-Assay wird beim Amersham-Assay die Konzentration an Autoantikörpern in "units" ausgedrückt (beim Farr-Assay in % -DNS-Bindung). Der Amersham-Assay wurde in zahlreichen klinischen Studien hinsichtlich seiner Validität bei der Entdeckung von Anti-DNS-Antikörpern beim systemischen Lupus erythematosus untersucht (Holian et al., 1975, Brit. J. Annal. Rheumat. Dis. 34 : 438; Kaye et al. Medical Journal 1977; Häcki and Grob DMW 101 : 1; Monier et al., 1978, Ann. Immunol. (Inst. Pasteur) ; 129 C: 463; Miller et al., 1980, J.Rheumatol. 7 : 660).

Von anderen Untersuchern wurde anstelle des Amersham-Assays, der auf [125]Jod-markierter E.coli-DNS beruht, eine $^{14}$C-markierte DNS von Amersham für einen Original-Farr-Assay oder eine modifizierte Form benutzt (Manthorpe et al., 1978, Allergy 33 : 42). In neuerer Zeit wurde anstelle von markierter E.coli-DNS auch markierte Phagen-DNS benutzt (Aarden et al., 1976, J. Immunol.Methods 11 : 153) oder Plasmid-DNS (Casperson and Foss, 1980, J.Immunol. Methods 36 : 293). Die Verwendung von Phagen- oder Plasmid-DNS erschien günstiger als die Verwendung von E.coli- oder Kalbsthymus-DNS, da Phagen- und Plasmid-DNS frei von Begleitproteinen ist, wie sie insbesondere Eukaryonten -DNS als festhaftende Proteinbestandteile aufweist, die sich durch biochemische Methoden nicht von der DNS entfernen lassen, wenn man nicht eine massive Verdauung mit Proteasen durchführen will. Hinter einer positiven Präzipitationsreaktion im Farr-Assay können sich demnach auch Antikörper gegen DNS-Proteinstrukturen verbergen.

Anstelle einer Fällung mit Ammoniumsulfat wurde von Smenk and Aarden (1980, J. Immunol. Methods 39 : 165) auch eine Fällung der Doppelstrang-DNS-Antikörperkonjugate durch Polyethylenglykol (PEG) vorgeschlagen. Mit dieser Methode werden auch schwach avide Anti-DNS-Antikörper mit erfaßt.

Wenngleich die Farr-Assay-Modifikationen, bei denen markierte Phagen- oder markierte Plasmid-DNS benutzt wird, noch am genauesten von allen bisher beschriebenen Nachweismethoden zu sein scheinen, liegt der gravierende Nachteil dieser Tests in der Verwendung von radioaktivem Material mit all seinen Problemen der Strahlengefährdung der Labormitarbeiter, der Umweltverschmutzung sowie der Schwierigkeiten beim Abtransport des radioaktiven Materials.

Der Amersham-Buchler Anti-Doppelstrang-DNS-Test ist mit dem großen Nachteil versehen, daß die DNS auch Einzelstrang-DNS-Anteile enthält. Wird die Amersham-Buchler [125]Jod-markierte E.coli-DNS mit S1-Nuklease behandelt, so wird anschließend eine deutliche Verminderung der präzipitierbaren Radioaktivität nach Inkubation mit hochpositiven Anti-DNS-Antikörper-Anteilen in den Seren festgestellt. Das Vorhandensein von Strangbrüchen und Einzelstrang-DNS-Anteilen in der Amersham-Buchler-DNS ist auch insoweit zu erwarten, als die $^{125}$Jod-Markierung und die nachfolgende Lagerung der DNS zu einer vorhersagbar zunehmenden Radiolyse der Produkte führen kann. Darüberhinaus enthält die Amersham-Buchler-DNS nach Aussage der Fa.Amersham Buchler einen Anteil von bis zu 10 Prozent Proteinen, von denen es nicht klar ist, ob sie nicht ebenfalls mit $^{125}$Jod-markiert vorliegen. Das Ergebnis kann daher auch Antikörper gegen Histone oder andere Kernbestandteile vortäuschen.

Ein Nachweis von Doppelstrang-DNS-Antikörpern kann auch durch eine sogenannte Millipore-Filtermethode erfolgen (Ginsberg und Keiser, 1973, Arthritis and Rheumatism 16 : 199). Dieser Assay basiert auf der Tatsache, daß Doppelstrang-DNS nicht an Millipore-Filter Typ HAWP bindet, daß jedoch Immunglobuline an diesem Filtertyp sehr stark binden. Bei dem Assay wird eine Serumprobe mit markierter Doppelstrang-DNS, beispielsweise T4-Bakteriophagen-DNS inkubiert und anschließend über den Millipore-Filter filtriert. Die dabei festhaftende Radioaktivität am Filter wird anschließend gemessen. Die Methode wurde von mehreren Autoren verwendet (Ludivico et al., 1980,J.Rheumatol. 7 : 843; Lewis et al., 1973, J. Immunol. Methods 3 : $^{14}$C-markierte E.coli-DNS und Absaugen über einen Glasfilter; Lentz et al., 1976, Arthritis and Rheumatism 19 : 867).

Der Filter-Assay ist kommerziell nicht erhältlich, ist sehr arbeitsaufwendig und hat überdies den Nachteil, daß wiederum, wie auch bei dem Farr-Assay, radioaktiv markierte DNS verwendet werden muß.

Eine weitere Nachweismethode von Anti-Doppelstrang-DNS-Antikörpern kann durch indirekte Immunfluoreszenz mit Crithidia luciliae als Substrat erfolgen.

Aarden und Mitarbeiter (1975, Ann.N.Y.Acad.Sci. 254 : 505) führten eine Methode zum indirekten Nachweis von Anti-DNS-Antikörpern durch indirekte Immunfluoreszenz ein, bei der als Substrat Crithidia luciliae, ein für Menschen nichtpathogener Hämoflagellat, benutzt wurde. Hierbei werden die einzelligen Crithidien auf Objektträger aufgebracht, aufgetrocknet und anschließend konsekutiv zuerst mit dem Patientenserum und darauffolgend mit einem FITC-markierten Anti-Humanimmunglobulin als Sekundärantikörper inkubiert. Entscheidend ist nicht die Anfärbung des Kernes, sondern die Anfärbung des Kinetoplasten, der histonfreie zirkuläre Doppelstrang-DNS enthält (Fouts et al., 1975, J.Cell.Biol. 67 : 378; Friou und Delain, 1969, Proc. Natl. Acad.Sci. USA 62 : 210; Steinart, 1965, Exp.Cell. Res. 39 : 69). Die Bedeutung des Tests zum Nachweis von Anti-Doppelstrang-DNS-Antikörpern beim systemischen Lupus erythematosus ist von zahlreichen Gruppen untersucht und mit der Sensitivität des Farr-Assays verglichen worden (Crowe et al., 1978, Arthritis and Rheumatism 21 : 309; Davis et al., 1977, J.Rheumatism 4 : 15; Deegan et al., 1978, Am. J. Clin. Path. 69 : 599; Sontheimer und Gillian, 1978, J.Lab.Clin.Meth. 91 : 505; Tourville and Benn, 1978, J. Clin. Microbiol. 7 : 219; Whiteside and Dixon, 1979, Am. Soc. Clin. Pathol. 72 : 829; Whitehouse et al., 1983, Z.Rheumatol. 42 : 28; Nilsson and Biberfeld, 1980, J.Clin. Lab. Immunol. 3 : 197). Der Crithidia lucilia IF-Test zum Nachweis von Anti-Doppelstrang-DNS-Antikörpern wird von der Fa. Zeus als Testkit angeboten.

Von anderer Seite ist anstelle von Crithidia lucilia die Verwendung von humanen Metaphasenchromosomen vorgeschlagen worden (Somerfield und Wilson, 1980, J.Clin.Lab. Immunol. 3 : 203).

Allen immunofluoreszenzmikroskopischen Untersuchungen ist der Nachteil inne, selbst bei Verwendung standardisierter FITC-markierter Sekundärantikörper (zum Nachweis der menschlichen an Kinetoplasten-DNS oder Metaphasenchromosomen-DNS gebundenen Immunoglobuline) und bei Einhaltung standardisierter Wasch- und Inkubationsbedingungen sehr stark von physikalischen Parametern der verwendeten Fluoreszenzmikroskope (Beleuchtungsart, Erregerfilter, Sperrfilter, dichroitischer Spiegel, Lampe, Alter der Lampe) abhängig zu sein. Darüber hinaus können mit dieser Methode nur Angaben in Titern (d.h. in Verdünnungen über 2) gemacht werden.

In der Literatur fehlt es nicht an Vorschlägen, Autoantikörper gegen Doppelstrang-DNS durch enzym-immunologische Methoden (ELISA) oder Radioimmunosorbenttests (RAST) nachzuweisen. Die ELISA-Methode wurde zuerst von Engvall und Perlmann,(l971, Immunochemistry 8 : 871) beschrieben. Im Prinzip basieren die veröffentlichten Tests darauf, daß das Antigen (Doppelstrang-DNS) an eine feste Phase (meist Mikrotiterplatten) gebunden werden, anschließend werden diese Antigen-beschichteten Platten mit Patientenserumverdünnungen inkubiert, wobei sich die Antikörper an das Antigen binden. Anschließend werden die gebundenen Antikörper durch enzymmarkierte Sekundärantikörper entdeckt und gemessen.

Das Anbinden von nativer Doppelstrang-DNS (S1-Nuklease-behandelte $^{14}$C-markierte native E.coli-DNS, Amersham Buchler) an Poly-l-Lysinbeschichtete Platten scheint noch die am wirksamsten von allen bis hierher beschriebenen Methoden zu sein. Es zeigte sich aber, daß Poly-l-Lysinbeschichtete Platten eine sehr starke unspezifische Bindung für Serum-IgG zeigen, wobei es sich dabei wahrscheinlich überwiegend um IgG mit niedrigem isoelektrischen Punkt handelt. Die starke unspezifische Bindung ließ sich auch nicht herabdrücken, wenn die mit Poly-l-Lysin und DNS beschichteten Platten anschließend mit Rinderserumalbumin oder anderen Proteinen nachträglich überschichtet wurden, oder wenn während der folgenden ELISA-Schritte verschiedene Additive zum Patientenserum oder dem Sekundärantikörper hinzugegeben wurden. Auch ein "Nachcoaten" mit Polyglutamat (Molekulargewicht 5 - 15 kD) vermochte die unspezifische Bindung von humanem Serum-IgG nicht vollständig zu unterdrücken. Beim Testen von Poly-l-Lysinen verschiedener Größe zeigte es sich, daß die Menge an gebundener Doppelstrang-DNS mit dem Molekulargewicht des verwendeten Poly-l-Lysins anstieg. Andererseits hatte jedoch das großmolekulare Poly-l-Lysins die höchste unspezifische Bindung.

Die Autoren Halbert et al.,(1981, J.Immunol.Clin.Meth. 97 : 97)banden die DNS direkt an Plastikscheiben, die Isothiocyanatgruppen enthielten. Die Einführung von Isothiocyanatgruppen basierte auf der Methode Halbert und Anken (1977, J. Infect. Dis. 136 : 318 ). Vermutlich basiert der von der Fa. Cordis Laboratories kommerziell vertriebene ELISA zum Nachweis von Doppelstrang-DNS-Antikörpern auf dieser Methode. Die Verwendung von methyliertem Rinderserumalbumin (BSA) brachte keine gute Bindung von markierter Doppelstrang-DNS an Plastikoberflächen. Die Menge an gebundener DNS war gegenüber einer Vorbeschichtung mit Protaminsulfat oder Poly-l-Lysin deutlich am schlechtesten. Es liegt bezüglich des Rinderserumalbumins sicher keine kovalente Bindung vor, da es eluierbar ist. Es kommt als gravierender Nachteil hinzu, daß Rinderserumalbumin für Tests mit Humanseren ungeeignet ist, da in den zu testenden Seren in unvoraussagbarer Weise Antikörper gegen Rinderserumalbumin vorliegen können, die sodann die Testergebnisse durch unerwünschte Bindungen verfälschen.

Protaminsulfat als Beschichtungs-Medium hat den entscheidenden Nachteil, auch Antikörper gegen Histone mit zu erfassen (Rubin et al., 1983, J. Immunol. Methods 63 : 359).

Nach eigenen Untersuchungen und Angaben von Engvall (Lancet, Dec. 1976, 25 : 1410) erscheint es als unwahrscheinlich, daß die Beschichtung von Mikrotiterplatten direkt mit Doppelstrang-DNS erfolgt. Bei verschiedenen Versuchen mit $^{14}$C-markierter S1-Nuklease behandelter E.coli-DNS (Amersham Buchler) und bei Verwendung unterschiedlicher Puffer während des Beschichtungsschrittes konnte keine nennenswerte Bindung von nativer DNS an verschiedene Plastikmikrotiterplatten (Polystyrol oder PVC) gemessen werden.

Es besteht somit der Verdacht, daß diejenigen Autoren, die eine direkte Beschichtung von Mikrotiterplatten mit Doppelstrang-DNS beschrieben haben, tatsächlich eine Beschichtung mit Einzelstrang-DNS oder zumindest einer Doppelstrang-DNS, die Einzelstrang-DNS-Abschnitte enthielt, vornahmen. Eine solche Antigenbeschichtung ist naturgemäß nicht in der Lage, Antikörper gegen Doppelstrang-DNS sensu strictu zu erkennen.

Der von der Fa.Cordis vertriebene ELISA zum Nachweis von Autoantikörpern gegen DNS vermag nach eigenen Angaben der Fa. Cordis nicht zwischen Einzelstrang-DNS und Doppelstrang-DNS unterscheiden. Dies kann möglicherweise in der Kupplungsmethode über Isothiocyanat begründet sein, das vorwiegend mit primären Aminogruppen reagiert, die in einer Doppelstrang-DNS in freier Form nahezu nicht vorhanden sind.

Die bisher veröffentlichten Arbeiten unterscheiden sich somit vor allen Dingen hinsichtlich der Methode, mit der Doppelstrang-DNS an die feste Phase gebunden wurde, und weisen die genannten Nachteile auf.

Es war daher Aufgabe der vorliegenden Erfindung, einen Immuntest zum Nachweis von Antikörpern gegen DNS zur Verfügung zu stellen, bei dem durch die Art der Bindung der DNS an eine feste Phase eine hochgradige Spezifität für Doppel- oder Einzelstrang-DNS sichergestellt wird.

Die Aufgabe wird dadurch gelöst, daß die Aktivierung durch Makromoleküle erfolgt, so daß die DNS kovalent gebunden wird.

Es werden somit feste Phasen zuerst mit Makromolekülen inkubiert und anschließend wird DNS durch eine kovalente Bindung an die Beschichtungsmoleküle gebunden. Die für die Beschichtung der festen Phasen verwendeten Makromoleküle, an die die DNS kovalent gebunden wird, bewirken eine möglichst geringe unspezifische Bindung von Immunglobulinen. Durch die Verwendung solcher Makromoleküle und nachträgliche kovalente Bindung von DNS kann nahezu jede feste Phase zum Anheften von Doppelstrang-DNS benutzt werden. Es liegt hier somit erstmalig die Verwendung einer kovalenten Bindung von DNS an eine feste Phase in Zusammenhang mit enzymimmunologischen und radioimmunologischen Nachweismethoden von Doppelstrang-DNS-Antikörpern oder Einzelstrang-DNS-Antikörpern vor.

Makromoleküle, die sehr leicht an feste Phasen binden, können bevorzugt verwendet werden.

Dies können beispielsweise Proteine sein.

Unter den Proteinen eigenen sich insbesondere Casein Gelatine oder Humanserumalbumin sehr gut. Diese Makromoleküle binden leicht an die feste Phase und sind problemlos erhältlich und weisen keinerlei Schwierigkeiten in ihrer Handhabung und Verarbeitung bei der Beschichtung der festen Phase auf.

Der neue Immuntest zeichnet sich insbesondere dadurch aus, daß wahlweise native Doppelstrang-DNS oder Einzelstrang-DNS kovalent gebunden werden kann und je nach Aufgabenstellung sehr spezifisch zwischen Antikörpern gegen Doppelstrang-DNS oder Einzelstrang-DNS unterschieden werden kann.

Die kovalente Bindung zwischen dem Makromolekül und der DNS kann durch Induktion mit Carbodiimidderivaten unterstützt und gefördert werden.

Um alle offenen Bindungsstellen abzudecken, die während der kovalenten Bindungsreaktion eventuell freigeworden sein könnten, empfiehlt es sich, die mit den Makromolekülen und der DNS beschichtete feste Phase noch einmal mit einer Lösung zu inkubieren, die dieselben Makromoleküle, wie sie für die Bindung an die feste Phase verwendet wurden, enthält.

Bevorzugt verwendete feste Phasen sind Mikrotiterplatten, Kunststoffröhrchen, Kunststoffkugeln oder auch Glasplatten.

Wenn die Versuchsanordnung die Verwendung von Filtern erforderlich macht, sind bevorzugt präaktivierte Filter zu verwenden.

Die, wie geschildert, beschichteten festen Phasen werden sodann zuerst mit den Lösungen inkubiert, die die nachzuweisenden Antikörper enthalten, also beispielsweise mit Seren von Patienten, sodann gewaschen und anschließend entweder mit enzym- oder radioaktiv-markierten Antikörpern gegen humane Immunoglobuline inkubiert.

Bei den genannten markierten Antikörpern kann es sich um monoklonale oder polyklonale Antikörper gegen humane Immunglobuline handeln.

Eine Verbesserung des Signal- Rausch-Verhältnisses des erfindungsgemäßen Immuntests kann sich durch Zugabe von Casein oder Gelatine oder Humanserumalbumin zu dem ersten und/oder zweiten Inkubationsschritt ergeben.

Bei Verwendung von enzymmarkierten Antikörpern werden die festen Phasen nach nochmaligem Waschen mit einer Substratlösung inkubiert, die dem verwendeten Enzym entspricht. Nach Abstoppen der Substratreaktion kann das Ausmaß der Substratreaktion in einem Photometer gemessen werden.

Bei Verwendung von radioaktiv markierten Antikörpern gegen Immunglobuline werden die festen Phasen nach Waschen entweder in einem Gammacounter zum Zählen gegeben, und zwar bei Verwendung von Gamma-imitierender Radionukleide als Marker für die Sekundärantikörper, oder zusammen mit Szintillationslösungen in einen Beta-Counter gegeben, wenn Beta-imitierende Radionukleide als Marker für die Sekundärantikörper verwendet werden.

Die Erfindung wird im folgenden anhand von Beispielen näher erläutert.

Beispiel 1

Mikrotiterplatten werden mit einer Lösung von 2% Casein (Sigma, München) in einem Carbonatpuffer (3,18 g $Na_2CO_3$, 5,86 g $NaHCO_3$, 0,4 $NaN_3$ pro 2 l, pH 9,66)mit je 250µl/ Plattenvertiefung inkubiert. Nach zweimaligem Waschen der Plattenvertiefungen mit destilliertem Wasser und einmaligem Waschen mit 0,1 M/ml Morpholinoethansulfonsäurepuffer pH 6.0 wird in jede Mikrotiterplattenvertiefung 200 µl hochreine, S1-Nuklease-behandelte Kalbsthymus-DNS (Sigma, München) in einer Konzentration von 10 µg/ml in 0,1 M/l Morpholinoethansulfonsäurepuffer pH 6.0 gegeben. Anschließend erfolgt eine Zugabe von 100 µg Carbodiimid (1-Cyclohexyl-3-(2-morpholinoethyl)carbodiimid metho 4-toluolsulfat (Serva)) in einer Konzentration von 100 mg/ml in $H_2O$. Die Platten werden für 1-16 h bei 4°C auf einer Drehscheibe inkubiert und anschließend dreimal mit 0,1 M/l Morpholinoethansulfonsäurepuffer pH 6.0 gewaschen. Anschließend werden in alle Plattenvertiefungen jeweils 300 µl 2%iges Casein in Carbonatpuffer (s.o.) gegeben und 2 h bei 37°C inkubiert. Die Platten werden dreimal mit Phosphat-gepufferter Saline (0,15 NaCl + 0,01 M/l $NaH_2PO_4$/$Na_2HPO_4$ pH 7,2)inkubiert. Die so vorbehandelten Platten können nach kurzem Waschen mit Aqua dest. gefriergetrocknet und unter Vakuum in Folien verschweißt werden. Sie sind monatelang haltbar.

Beispiel 2

Durchführung des Nachweises von Anti-Doppelstrang-DNS-Antikörpern in Patientenseren unter Verwendung von Mikrotiterplatten, wie sie im Beispiel 1 hergestellt wurden.

Zur Durchführung des Tests werden die Seren 1:50 bzw. 1:100 mit PBS oder PBS-0,5% Tween® 20 verdünnt und jeweils 150 µl in die Mikrotiterplattenvertiefungen eingefüllt. Nach Inkubation für zwei Stunden bei 37°C werden die Platten dreimal mit PBS, das zusätzlich 0,05% TWEEN 20 enthält, gewaschen, dann folgt eine Zugabe von jeweils 150 µl alkalischphosphatase-markierten affinitätschromatographisch gereinigten Fab-2- Fragmenten von Ziegen-Anti-Human-IgG (Fc)-Antikörpern (Dianova-Jackson, Hamburg), die 1:1000 mit PBS verdünnt werden. Nach einer zweistündigen Inkubation bei 37°C werden die Platten dreimal mit PBS-TWEEN und dreimal mit PBs gewaschen. Anschließend erfolgt eine Zugabe von jeweils 150 µl der Substratlösung (1 mg/ml p-Nitrophenylphosphat in 0, 1 M/l Diethanolaminpuffer pH 8,0, zusätzlich 1 mM/MgCl₂ und 0,1 mM/l ZnCl₂ enthaltend) in alle Mikrotiterplattenvertiefungen. Die Reaktion wird gestoppt durch Zugabe von jeweils 50 µl einer 0,2 M/l Ethylendiamintetraessigsäurenatriumsalzlösung in alle Mikrotiterplattenvertiefungen. Die Substratreaktion kann in einem Mikrotiterplattenphotometer etwa Dynatech MR600 oder einem ähnlichen Photometer bei einer Wellenlänge von 406 nm gemessen werden.

Ausgedehnte Vorversuche zeigten, daß die Extinktionswerte von etwa 50 gesunden Blutspendern bei 0,06 + 0,02 (SD) lagen. Bei Verwendung von Seren von Patienten mit Antikörpern gegen Doppelstrang-DNS (soweit dies durch den Amersham Buchler Test nachweisbar ist) wurden Extinktionen von > 2,0 beobachtet. Unter Zugrundelegung einer Normalgrenze (Mittelwert der Normalpersonen + 2 (SD) einer Extinktion von 0,1 lagen also die Signal-Rausch-Verhältnisse bei einem Faktor >20.

Analog zum angegebenen Verfahren kann die Menge an bindenden Antikörpern aber auch durch eine Zweitinkubation mit ¹²⁵Jod-markierten Antikörpern gegen humane Immunglobuline und Messung in einem Gammacounter als RIA durchgeführt werden.

Überraschend und nicht vorhersehbar führte das erfindungsgemäße Verfahren der kovalenten Bindung von Doppelstrang-DNS an adsorptiv gebundene Moleküle, die selbst nur eine geringe unspezifische Bindung von Immunglobulinen bewirken, also

- zu einer reproduzierbaren und hohen Bindung von Doppelstrang-DNS an die zur Verfügung stehende benetzte Fläche (bei Mikrotiterplatten mit Vertiefungen mit 7 mm Bodendurchmesser und einer Füllung mit 150 µl ca. 125 mm² Fläche) und
- zu einem gegenüber dem Farr-Assay-Prinzip erheblich erweiterten Meßbereich bei maximalen Signal-Rausch-Verhältnissen von größer als 20.

Durch die Möglichkeit,feste Phasen, wie Mikrotiterplatten oder Kunststoffkugeln, unter standardisierten Bedingungen und nach dem erfindungsgemäßen Verfahren mit einer konstanten Menge von Doppelstrang-DNS oder Einzelstrang-DMS zu beschichten, ergibt sich die Möglichkeit, kommerziell vermarktbare Testsysteme zum Nachweis von Antikörpern gegen Doppelstrang-DNS oder Einzelstrang-DNS zu entwickeln.

**Ansprüche**

1. Verfahren zur Bindung von DNS, vorzugsweise Doppelstrang-DNS, zum Nachweis von gegen diese DNS gerichteten Autoantikörpern im Rahmen eines Immuntestes, dadurch gekennzeichnet,
daß eine feste Phase zuerst mit Makromolekülen inkubiert wird und
daß anschließend die DNS durch eine kovalente Bindung an die Beschichtungsmoleküle bildenden Makromoleküle gebunden wird.

2. Immuntest nach Anspruch 1, dadurch **gekennzeichnet**, daß als Makromoleküle solche verwendet werden, die leicht an feste Phasen binden.

3. Immuntest nach Anspruch 1 und/oder Anspruch 2, dadurch **gekennzeichnet**, daß als Makromoleküle Proteine verwendet werden.

4. Immuntest nach Anspruch 3, dadurch **gekennzeichnet**, daß als Protein Casein oder Gelatine oder Humanserumalbumin verwendet wird.

5. Immuntest nach mindestens einem der vorhergehenden Ansprüche, dadurch **gekennzeichnet**, daß native Doppelstrang-DNS oder Einzelstrang-DNS kovalent gebunden wird.

6. Immuntest nach mindestens einem der vorhergehenden Ansprüche, dadurch **gekennzeichnet**, daß zur Induktion der kovalenten Bindung zwischen dem Makromolekül und der DNS Carbodiimidderivate verwendet werden.

7. Immuntest nach mindestens einem der vorhergehenden Ansprüche, dadurch **gekennzeichnet**, daß die an die Makromoleküle kovalent gebundene DNS nachträglich mit denselben Makromolekülen überschichtet wird.

8. Immuntest nach mindestens einem der vorhergehenden Ansprüche, dadurch **gekennzeichnet**, daß als feste Phase Kunststoff, Glas oder Zellulose verwendet wird.

9. Immuntest nach mindestens einem der Ansprüche 1 bis 7, dadurch **gekennzeichnet**, daß als feste Phase präaktivierte Filter verwendet werden.

10. Immuntest nach einem der vorhergehenden Ansprüche, dadurch **gekennzeichnet**, daß die mit den Makromolekülen und der an diese kovalent gebundenen DNS beschichtete feste Phase erst mit einem Serum, das den nachzuweisenden Antikörper enthält und sodann mit enzym- oder radioaktiv markierten Antikörpern gegen humane Immunglobuline inkubiert wird.

**11.** Immuntest nach Anspruch 10, dadurch **gekennzeichnet**, daß die markierten Antikörper monoklonale oder polyklonale Antikörper sind.

**12.** Immuntest nach Anspruch 10 und/oder 11, dadurch **gekennzeichnet**, daß zu dem Serum und den markierten Antikörpern Casein, Gelatine oder Humanserumalbuminzusätze zugefügt werden.

## Claims

**1.** Process for the bonding of DNA, preferably double-strand DNA, for the detection of auto-antibodies directed against said DNA within the framework of an immune test, characterized in that a solid phase is firstly incubated with macromolecules and subsequently the DNA is bound by a covalent bond to the macromolecules forming the coating molecules.

**2.** Immune test according to claim 1, characterized in that the macromolecules used can be easily bound to solid phases.

**3.** Immune test according to claims 1 and/or 2, characterized in that proteins are used as macromolecules.

**4.** Immune test according to claim 3, characterized in that casein or gelatin or human serum albumin is used as the protein.

**5.** Immune test according to at least one of the preceding claims, characterized in that native double-strand DNA or single-strand DNA is covalently bonded.

**6.** Immune test according to at least one of the preceding claims, characterized in that for inducing covalent bonding between the macromolecule and the DNA use is made of carbodiimide derivatives.

**7.** Immune test according to at least one of the preceding claims, characterized in that the DNA covalently bonded to the macromolecules is subsequently coated with the same macromolecules.

**8.** Immune test according to at least one of the preceding claims, characterized in that plastic, glass or cellulose is used as the solid phase.

**9.** Immune test according to at least one of the claims 1 to 7, characterized in that preactivated filters are used as the solid phase.

**10.** Immune test according to one of the preceding claims, characterized in that the solid phase coated with the macromolecules and the DNA covalently bonded thereto is firstly incubated with a serum, which contains the antibodies to be detected and then with enzymatically or radioactively labelled antibodies against human immunoglobulins.

**11.** Immune test according to claim 10, characterized in that the labelled antibodies are monoclonal or polyclonal antibodies.

**12.** Immune test according to claims 10 and/or 11, characterized in that casein, gelatin or human serum albumin additives are added to the serum and the labelled antibodies.

## Revendications

**1.** Procédé pour lier de l'A.D.N., et de préférence de l'A.D.N. à double hélice, afin de détecter des autoanticorps dirigés contre cet A.D.N. dans le cadre d'un test immunologique, caractérisé par le fait que l'on incube tout d'abord une phase solide avec des macromolécules et qu'on lie ensuite l'A.D.N. par une liaison covalente aux macromolécules qui forment les molécules de revêtement.

**2.** Test immunologique selon la revendication 1, caractérisé par le fait que l'on utilise comme macromolécules celles qui se lient facilement à des phases solides.

**3.** Test immunologique selon la revendication 1 et/ou selon la revendication 2, caractérisé par le fait que l'on utilise des protéines comme macromolécules.

**4.** Test immunologique selon la revendication 3, caractérisé par le fait que l'on utilise comme protéines de la caséine ou de la gélatine ou de la sérumalbumine humaine.

**5.** Test immunologique selon l'une au moins des revendications précédentes, caractérisé par le fait qu'on lie par covalence de l'A.D.N. native à double hélice ou de l'A.D.N. native à simple hélice.

**6.** Test immunologique selon l'une au moins des revendications précédentes, caractérisé par le fait que l'on utilise des dérivés du carbodiimide pour induire la liaison covalente entre la macromolécule et l'A.D.N.

7. Test immunologique selon l'une au moins des revendications précédentes, caractérisé par le fait que l'on recouvre postérieurement l'A.D.N. lié de manière covalente aux macromolécules, et ce, au moyen des mêmes macromolécules.

8. Test immunologique selon l'une au moins des revendications précédentes, caractérisé par le fait que l'on utilise comme phase solide de la matière plastique, du verre ou de la cellulose.

9. Test immunologique selon l'une au moins des revendications 1 à 7, caractérisé par le fait que l'on utilise comme phase solide des filtres activés au préalable.

10. Test immunologique selon l'une au moins des revendications précédentes, caractérisé par le fait que l'on incube la phase solide revêtue des macromolécules et de l'A.D.N. qui est lié à celles-ci de manière covalente, tout d'abord avec un sérum qui contient l'anticorps à détecter, et ensuite avec des anticorps anti-immunoglobuline humaine qui sont marqués par voie enzymatique ou radioactive.

11. Test immunologique selon la revendication 10, caractérisé par le fait que les anticorps marqués sont des anticorps monoclonaux ou polyclonaux.

12. Test immunologique selon la revendication 10 et/ou 11. caractérisé par le fait que l'on ajoute au sérum et aux anticorps marqués de la caséine, de la gélatine ou des additifs de sérumalbumine humaine.